(19) Europäisches Patentamt
European Patent Office
Office européen des brevets

(11) Publication number: **0 526 936 A2**

(12) # EUROPEAN PATENT APPLICATION

(21) Application number: **92202300.7**

(22) Date of filing: **25.07.92**

(51) Int. Cl.$^5$: **C07C 235/76**, C07D 209/20, A61K 31/195, A61K 31/405, C07C 211/10, C07C 215/12, C07C 279/14

The applicant has filed a statement in accordance with Rule 28 (4) EPC (issue of a sample only to an expert). Accession number(s) of the deposit(s): ATCC 74084

(30) Priority: **02.08.91 US 739950**
**02.08.91 US 739758**
**02.08.91 US 739932**
**09.07.92 US 907730**

(43) Date of publication of application:
**10.02.93 Bulletin 93/06**

(84) Designated Contracting States:
**CH DE FR GB IT LI NL**

(71) Applicant: **MERCK & CO. INC.**
**126, East Lincoln Avenue P.O. Box 2000**
**Rahway New Jersey 07065-0900(US)**

(72) Inventor: **Meinz, Maria Sandrino**
**24 Abbott Road**
**Somerset, NJ 08873(US)**
Inventor: **Pelaez, Fernando**
**C/Camino de Valderribas 10**
**E-28038 Madrid(ES)**
Inventor: **Omstead, Mary Nallin**
**13 Deer Path**
**Gladstone, NJ 07934(US)**
Inventor: **Milligan, James A.**
**8 Parkway Place**
**Parlin, NJ 08859(US)**
Inventor: **Diez, Maria Teresa**
**C/Olid, 4**
**E-28010 Madrid(ES)**
Inventor: **Onishi, Janet C.**
**350 Cherry Hill Road**
**Mountainside, NJ 07092(ES)**
Inventor: **Bergstrom, James D.**
**60 Rohill Road**
**Neshanic Station, NJ 08853(US)**
Inventor: **Jenkins, Rosalind F.**
**81 Lafayette Avenue**
**Somerset, NJ 08873(US)**
Inventor: **Harris, Guy H.**
**308 Shadowlawn Way**
**Cranford, NJ 07016(US)**
Inventor: **Jones, E. Tracy Turner**
**805 Highland Drive**
**Solona Beach, California 92075(US)**
Inventor: **Huang, Leeyuan**
**33 Will Lane**
**Watchung, NJ 07060(US)**
Inventor: **Kong, Yu Lin**
**57 Bryant Avenue**
**Edison, NJ 08820(US)**
Inventor: **Lingham, Russell B.**
**5 Johanna Lane**
**Watchung, NJ 07060(US)**
Inventor: **Zink, Deborah**
**37 Blenheim Road**
**Manalapan, NJ 07726(US)**

(74) Representative: **Barrett-Major, Julie Diane et al**
**Merck & Co., Inc. European Patent Department Terlings Park Eastwick Road**
**Harlow Essex CM20 2OR(GB)**

(54) **Cholesterol-lowering agents.**

(57) This invention relates to compounds of structural formula (I):

EP 0 526 936 A2

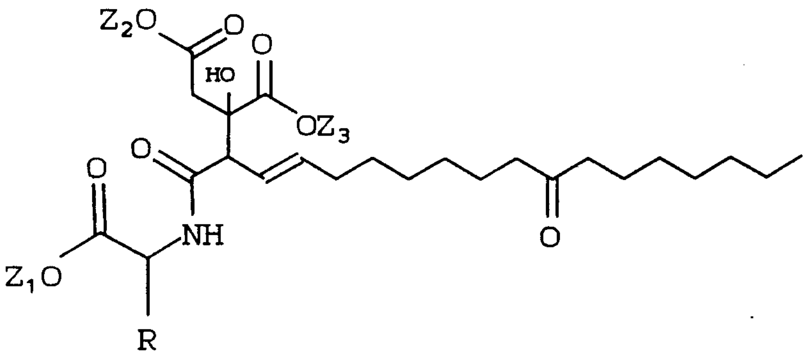

(I)

which are squalene synthase inhibitors and thus useful as cholesterol lowering agents, and antifungal agents. These compounds are also inhibitors of farnesyl protein transferase and farnesylation of the oncogene protein Ras and thus useful in treating cancer. This invention also relates to a process for obtaining compounds of structural formula (I).

BACKGROUND OF THE INVENTION

Hypercholesterolemia is known to be one of the prime risk factors for ischemic cardiovascular disease, such as arteriosclerosis. Bile acid sequestrants have been used to treat this condition; they seem to be moderately effective but they must be consumed in large quantities, i.e. several grams at a time and they are not very palatable.

MEVACOR® (lovastatin) and ZOCOR® (simvastatin), now commercially available, are members of a group of very active antihypercholesterolemic agents that function by limiting cholesterol biosynthesis by inhibiting the enzyme HMG-CoA reductase.

Squalene synthase (also called squalene synthetase) is the enzyme involved in the first committed step of the de novo cholesterol biosynthetic pathway. This enzyme catalyzes the reductive dimerization of two molecules of farnesyl pyrophosphate to form squalene. The inhibition of this committed step to cholesterol should leave unhindered biosynthetic pathways to ubiquinone, dolichol and isopentenyl t-RNA.

Previous efforts at inhibiting squalene synthase have employed pyrophosphate or pyrophosphate analog containing compounds such as those described in P. Ortiz de Montellano et al., J. Med. Chem. 20, 243 (1977), E.J. Corey and R. Volante, J. Am. Chem. Soc., 98, 1291 (1976), and U.S. Patent 5,025,003 to S. Biller. U.S. Patent 4,871,721 to S. Biller describes isoprenoid(phosphinylmethyl) phosphonates as inhibitors of squalene synthetase.

U.S. Patents 5,096,923; 5,026,554; and 5,102,907 and U.S. Patent Applications Serial Numbers 496,734 filed March 21, 1990, and 698,766 filed May 10, 1991 disclose non-phosphorus-containing substituted 2,8-dioxabicyclo[3.2.1]octane derivatives useful as squalene synthase inhibitors and U.S. Patent Application Serial No. 701,922 filed May 17, 1991, describes substituted quinuclidinyl oxadiazoles useful as squalene synthetase inhibitors. Furthermore, J. Antibiotics 45: 639-658 (1992) describe called squalestatin.

Recently it has been shown that certain natural product nonphosphorous containing inhibitors of squalene synthase and their esters are useful in inhibiting fungal growth. This utility is described in U.S. Pat No. 5,026,554.

The present invention is directed to the use of compounds of structural formula (I) which are squalene synthase inhibitors for the inhibition of fungal growth.

The present invention is also directed to the use of compounds of structural formula (I) which are inhibitors of farnesyl protein trnasferase for inhibition of farnesylation of the oncogene protein Ras and the treatment of cancer.

The Ras gene is found activated in many human cancers, including colorectal carcinoma, exocrine pancreatic carcinoma, and myeloid leukemias. Biological and biochemical studies of Ras action indicate that Ras functions like a G-regulatory protein, since Ras must be localized in the plasma membrane and must bind with GTP in order to transform cells (Gibbs, J. et al., Microbiol. Rev. 53:171-286 (1989). Forms of Ras in cancer cells have mutations that distinguish the protein from Ras in normal cells.

At least 3 post-translational modifications are involved with Ras membrane localization, and all 3 modifications occur at the C-terminus of Ras. The Ras C-terminus contains a sequence motif termed a "CAAX" or "Cys-Aaa$^1$-Aaa$^2$-Xaa" box (Aaa is an aliphatic amino acid, the Xaa is any amino acid) (Willumsen et al., Nature 310:583-586 (1984)). Other proteins having this motif include the Ras-related GTP-binding proteins such as Rho, fungal mating factors, the nuclear lamins, and the gamma subunit of transducin.

Farnesylation of Ras by the isoprenoid farnesyl pyrophosphate (FPP) occurs in vivo on Cys to form a thioether linkage (Hancock et al., Cell 57:1167 (1989); Casey et al., Proc. Natl. Acad. Sci. USA 86:8323 (1989)). In addition, Ha-Ras and N-Ras are palmitoylated via formation of a thioester on a Cys residue near a C-terminal Cys farnesyl acceptor (Gutierrez et al., EMBO J. 8:1093-1098 (1989); Hancock et al., Cell 57:1167-1177 (1989)). Ki-Ras lacks the palmitate acceptor Cys. The last 3 amino acids at the Ras C-terminal end are removed proteolytically, and methyl esterification occurs at the new C-terminus (Hancock et al., ibid). Fungal mating factor and mammalian nuclear lamins undergo identical modification steps (Anderegg et al., J. Biol. Chem. 263:18236 (1988); Farnsworth et al., J. Biol. Chem. 264:20422 (1989)).

Inhibition of Ras farnesylation in vivo has been demonstrated with lovastatin (Merck & Co., Rahway, NJ) and compactin (Hancock et al., ibid; Casey et al., ibid; Schafer et al., Science 245:379 (1989)). These drugs inhibit HMG-CoA reductase, the rate limiting enzyme for the production of polyisoprenoids and the farnesyl pyrophosphate precursor. It has been shown that a farnesyl-protein transferase using farnesyl pyrophosphate as a precursor is responsible for Ras farnesylation. (Reiss et al., Cell, 62:81-88 (1990); Schaber et al., J. Biol. Chem., 265:14701-14704 (1990); Schafer et al., Science, 249:1133-1139 (1990); Manne et al., Proc. Natl. Acad. Sci. USA, 87:7541-7545 (1990)).

Inhibition of farnesyl-protein transferase and, thereby, of farnesylation of the Ras protein, blocks the

ability of Ras to transform normal cells to cancer cells. Surprisingly, the compounds of the invention inhibit Ras farnesylation and, thereby, generate soluble Ras which, as indicated infra, can act as a dominant negative inhibitor of Ras function. While soluble Ras in cancer cells can become a dominant negative inhibitor, soluble Ras in normal cells would not be an inhibitor.

A cytosol-localized (no Cys-$Aaa^1$-$Aaa^2$-Xaa box membrane domain present) and activated (impaired GTPase activity, staying bound to GTP) form of Ras acts as a dominant negative Ras inhibitor of membrane-bound Ras function (Gibbs et al., Proc. Natl. Acad. Sci. USA 86:6630-6634 (1989)). Cytosol-localized forms of Ras with normal GTPase activity do not act as inhibitors. Gibbs et al., ibid, showed this effect in Xenopus octyes and in mammalian cells.

Administration of compounds of the invention to block Ras farnesylation not only decreases the amount of Ras in the membrane but also generates a cytosolic pool of Ras. In tumor cells having activated Ras, the cytosolic pool acts as another antagonist of membrane-bound Ras function. In normal cells having normal Ras, the cytosolic pool of Ras does not act as an antagonist. In the absence of complete inhibition of farnesylation, other farnesylated proteins are able to continue with their functions.

Farnesyl-protein transferase activity may be reduced or completely inhibited by adjusting the compound dose. Reduction of farnesyl-protein transferase enzyme activity by adjusting the compound dose would be useful for avoiding possible undesirable side effects such as interference with other metabolic processes which utilize the enzyme.

These compounds are inhibitors of farnesyl-protein transferase. Farnesyl-protein transferase utilizes farnesyl pyrophosphate to covalently modify the Cys thiol group of the Ras CAAX box with a farnesyl group. Inhibition of farnesyl pyrophosphate biosynthesis by inhibiting HMG-CoA reductase blocks Ras membrane localization in vivo and inhibits Ras function. Inhibition of farnesyl-protein transferase is more specific and is attended by fewer side effects then is the case for a general inhibitor of isoprene biosynthesis.

Previously, it has been demonstrated that tetrapeptides with the CAAX sequence inhibit Ras farnesylation (Schaber et al., ibid: Reiss et. al., ibid; Reiss et al., PNAS, 88:732-736 (1991)). However, the reported inhibitors of farnesyl-transferase are metabolically unstable or inactive in cells.

Pharmaceutical compositions containing the compounds of this invention and methods of treatment utilizing these compositions for use in inhibiting farnesyl-protein transferase and farnesylation of the oncogene protein Ras are described herein.

The present invention provides nonphosphorus containing inhibitors of squalene synthase.

## DETAILED DESCRIPTION OF THE INVENTION

The present invention is directed to novel compounds of structural formula (I) which are squalene synthase inhibitors:

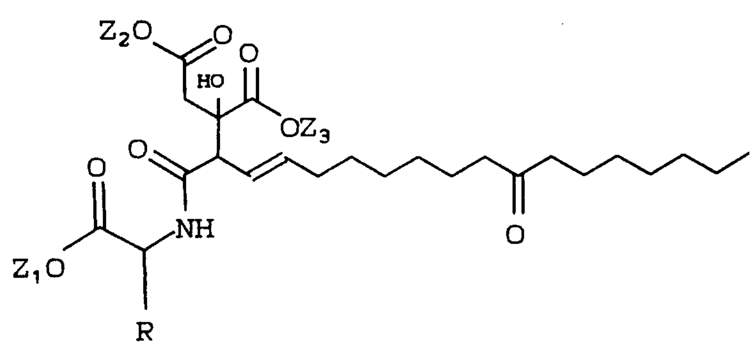

(I)

wherein
R is:

a) 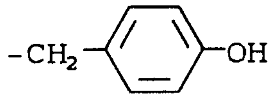 ,

b) 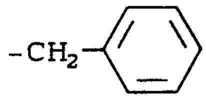 , or

c) 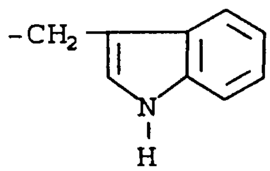 ; and

$Z_1$, $Z_2$ and $Z_3$ are independently:

- a) H;
- b) $C_{1-5}$alkyl; or
- c) $C_{1-5}$alkyl substituted with
  - i) phenyl, or
  - ii) phenyl substituted with methyl, methoxy, halogen (Cl, Br, F, I) or hydroxy;

or a pharmaceutically acceptable salt of a compound of formula (I).

One sub-class of compounds are those in which R is p-hydroxybenzyl. Exemplifying this sub-class are those compounds wherein $Z_1$, $Z_2$ and $Z_3$ are each hydrogen or a pharmaceutically acceptable salt thereof. The compound wherein R is p-hydroxybenzyl and $Z_1$, $Z_2$ and $Z_3$ are each hydrogen is hereafter referred to as Compound A.

Further illustrating this sub-class are those compounds in which R is p-hydroxybenzyl and in which one or more of $Z_1$, $Z_2$ or $Z_3$ is $C_{1-5}$alkyl or $C_{1-5}$alkyl substituted with phenyl or substituted phenyl wherein the substituent is methyl, methoxy, halogen or hydroxy. In a specific illustration, R is p-hydroxybenzyl and $Z_1$, $Z_2$ and $Z_3$ are each methyl. This compound is hereafter referred to as Compound B.

Another subclass are those compounds in which R is benzyl. Exemplifying this subclass are those compounds wherein R is benzyl and $Z_1$, $Z_2$ and $Z_3$ are each hydrogen or a pharmaceutically acceptable salt thereof. The compound wherein R is benzyl and $Z_1$, $Z_2$ and $Z_3$ are each hydrogen is hereafter referred to as Compound C.

Further illustrating this subclass are those compounds in which R is benzyl and in which one or more of $Z_1$, $Z_2$ or $Z_3$ is $C_{1-5}$ alkyl or $C_{1-5}$ alkyl substituted with phenyl or substituted phenyl wherein the substituent is methyl, methoxy, halogen or hydroxy.

A third subclass are those compounds in which R is $-CH_2$-3-indolyl and $Z_1$, $Z_2$, and $Z_3$ are each hydrogen or a pharmaceutically acceptable salt thereof. The compound wherein R is $-CH_2$-3-indolyl and $Z_1$, $Z_2$ and $Z_3$ are each hydrogen is hereafter referred to as Compound D.

Further illustrating this subclass are those compounds in which R is $-CH_2$-3-indolyl and in which one or more of $Z_1$, $Z_2$ or $Z_3$ is $C_{1-5}$ alkyl or $C_{1-5}$ alkyl substituted with phenyl or substituted phenyl wherein the substituent is methyl, methoxy, halogen or hydroxy.

The compounds of formula (I) are prepared in an aerobic fermentation procedure employing a novel fungal culture, MF5628, identified as Trichoderma viride Pers. or a mutant thereof. A mutant refers to an organism in which some gene on the genome is modified, leaving functional and heritable the gene or genes responsible for the organism's ability to produce the compounds of formula (I) in recoverable amounts. Trichoderma viride is a common and geographically widespread organism, present in many soils and organic substrata throughout the world.

The culture MF5628 is that of a fungus, Trichoderma viride Pers., isolated from soil collected in Kolonia, Pohnpei (formerly Ascension Island), Federated States of Micronesia. This culture has been deposited with the American Type Culture Collection at 12301 Parklawn Drive, Rockville, MD 20852 as ATCC 74084 on July 30, 1991, under the conditions of the Budapest Treaty.

Within the genus Trichoderma, this strain can be assigned to Trichoderma viride species aggregate

(Rifai, M. A. 1969. A revision of the genus Trichoderma. CMI Mycological Paper 116: 1-56) because it lacks sterile terminal appendages on the conidiophores, the phialides are long, tapered and arranged in a regular, dendritic pattern, and the conidia are, for the most part, subglobose and roughened.

The culture MF5628, identified as Trichoderma viride exhibits the following morphological features.

Colonies growing rapidly on most standard mycological media. On corn meal agar, malt-extract agar, or oatmeal agar reaching 40-45 mm in 48 hours at 27° C and covering a 9 cm plate in about five days. At first, colonies on malt-extract agar hyaline to white, sparse to loosely floccose, at maturity becoming dark yellowish green to dark green because of conidial production, Dark Green, Dark Yellowish Green, reverse olive yellow to Light Yellowish Olive (capitalized color names from Ridgway,R.,1912. Color Standards and Nomenclature, Washington, D.C.). On corn meal agar, tufts of conidiophores forming concentric rings, alternating with zones of hyaline, hyphae, on malt-extract and oatmeal agars, conidiophores develop uniformly over the agar surface.

Mycelium hyaline, septate, smooth-walled, 2.6-3.8 µm wide, forming chlamydospores which are globose to subglobose, smooth, 5.7-9.5 µm diameter. Conidiophores arising from surface of colony, often aggregated in pulvinate conidial pustules, profusely branched at wide angles, either singly or in groups of two or three in opposite or whorled arrangement, with branches decreasing in length towards the apex, terminating with phialides. Conidiogenous cells enteroblastic, phialidic, narrowly flask-shaped, slender, tapered at the apices, 5.7-9.5 x 2 µm, straight or slightly curved, single, or in groups of two or three, at wide angles to the conidiophore branch, forming a regular dendritic pattern, never together in dense aggregations. Conidia subglobose to ovoid 3.0-4.5 x 2.6-3.0 µm, pale green in KOH, slightly roughened, accumulating as a cluster at tips of the phialides.

Compounds of this invention can be obtained by culturing the above-noted microorganism in an aqueous nutrient medium containing sources of assimilable carbon and nitrogen, preferably under aerobic conditions. Nutrient media may also contain mineral salts and defoaming agents.

The preferred sources of carbon in the nutrient medium are carbohydrates such as glucose, glycerin, starch, dextrin, and the like. Other sources which may be included are maltose, mannose, sucrose, and the like. In addition, complex nutrient sources such as oat flour, corn meal, millet, corn and the like may supply utilizable carbon. The exact quantity of the carbon source which is used in the medium will depend, in part, upon the other ingredients in the medium, but is usually found in an amount ranging between 0.5 and 5 percent by weight. These carbon sources can be used individually in a given medium or several sources in combination in the same medium.

The preferred sources of nitrogen are amino acids such as glycine, methionine, proline, threonine and the like, as well as complex sources such as yeast extracts (hydrolysates, autolysates), dried yeast, tomato paste, soybean meal, peptone, corn steep liquor, distillers solubles, malt extracts and the like. Inorganic nitrogen sources such as ammonium salts (eg. ammonium nitrate, ammonium sulfate, ammonium phosphate, etc.) can also be used. The various sources of nitrogen can be used alone or in combination in amounts ranging between 0.2 to 70 percent by weight of the medium.

The carbon and nitrogen sources are generally employed in combination, but need not be in pure form. Less pure materials which contain traces of growth factors, vitamins, and mineral nutrients may also be used. Mineral salts may also be added to the medium such as (but not limited to) calcium carbonate, sodium or potassium phosphate, sodium or potassium chloride, magnesium salts, copper salts, cobalt salts and the like. Also included are trace metals such as manganese, iron, molybdenum, zinc, and the like. In addition, if necessary, a defoaming agent such as polyethylene glycol or silicone may be added, especially if the culture medium foams seriously.

The preferred process for production of compounds of this invention consists of inoculating spores or mycelia of the producing organism into a suitable medium and then cultivating under aerobic conditions.

Compounds of formula (I) may be isolated from the aerobic fermentation of a culture of MF5628 (ATCC 74084). A culture of MF5628 (ATCC 74084) is defined as substantially free of its natural soil contaminants and capable of forming compounds of structural formula (I) in recoverable amounts. A biologically pure culture of MF5628 (ATCC 74084) may also be employed.

The fermentation procedure generally is to first inoculate a preserved source of culture into a nutrient seed medium and to obtain, sometimes through a two-step process, growth of the organisms which serve as seeds in the production of the active compounds. After inoculation, the flasks are incubated with agitation at temperatures ranging from 20 to 30° C, preferably 25 to 28° C. Agitation rates may range up to 400 rpm, preferably 200 to 220 rpm. Seed flasks are incubated over a period of 2 to 10 days, preferably 2 to 4 days. When growth is plentiful, usually 2 to 4 days, the culture may be used to inoculate production medium flasks. A second stage seed growth may be employed, particularly when going into larger vessels. When this is done, a portion of the culture growth is used to inoculate a second seed flask incubated under similar

conditions but employing shorter time.

After inoculation, the fermentation production medium is incubated for 3 to 30 days, preferably 6 to 22 days, with or without agitation (depending on whether liquid or solid fermentation media are employed). The fermentation is conducted aerobically at temperatures ranging from 20 to 40° C. If used, agitation may be at a rate of 200 to 400 rpm. To obtain optimum results, the temperatures are in the range of 22 to 28° C, most preferably 24 to 26° C. The pH of the nutrient medium suitable for producing the active compounds is in the range of 3.5 to 8.5, most preferably 5.0 to 7.5. After the appropriate period for production of the desired compound, fermentation flasks are harvested and the active compound isolated.

An alcoholic or oxygenated solvent, such as an ester or ketone, is employed to extract a compound of this invention from the solid fermentation medium. The preferred solvent for extraction of the solid fermentation is methyl ethyl ketone (MEK). The mixture of the solvent and fermentation broth is vigorously stirred and filtered. The resulting MEK layer is washed with hexanes and concentrated to dryness in vacuo for anion exchange chromatography. The preferred resin is BioRad AG4-X4 (acetate). The column is washed with a 6:4 acetonitrile ($CH_3CN$):water and the compound is eluted with an acidic solution. The preferred eluent is 0.16 N sulfuric acid in 6:4 $CH_3CN$:water. The eluent is extracted with ethyl acetate and concentrated to yield the crude compound.

Further purification is accomplished using reversed-phase high pressure liquid chromatography (RP-HPLC). The preferred adsorbent for this chromatography is a octadecylsilane bonded phase silica gel. The preferred eluant for RP-HPLC is a mixture of acetonitrile and water buffered at low pH, such as 0.1% phosphoric or trifluoroacetic acid.

Further purification may also be accomplihsed using high speed countercurrent chromatography. The preferred solvent system is a mixture of hexanes: ethyl acetate: methanol: 0.1% aqueous phosphoric acid (5:5:5:5). The preferred apparatus is the ITO multilayer-coil manufactured by P.C. Inc., Potomac, Maryland, USA; however, other ocuntercurrent chromatography equipment may be used.

Esters of Compound A, C or D may be prepared by dissolving Compound A, C or D in a dry organic solvent, preferably tetrahydrofuran (THF) at 0-30° C and treating with the appropriately substituted isourea for 8-24 hours, cooling to -15° C and filtering the urea. The mono-, di- and tri- esters may be prepared by varying the number of equivalents of isourea used. The filtrate is concentrated under reduced pressure to yield the desired ester.

The present invention is also directed to a method of inhibiting cholesterol biosynthesis which comprises the administration to a subject in need of such treatment a nontoxic therapeutically effective amount of a compound represented by structural formula (I) and pharmaceutically acceptable salts thereof. Specifically, the compounds of this invention are useful as antihypercholesterolemic agents for the treatment of arteriosclerosis, hyperlipidemia, familial hypercholesterolemia and the like diseases in humans. They may be administered orally or parenterally in the form of a capsule, a tablet, an injectable preparation or the like. It is usually desirable to use the oral route. Doses may be varied, depending on the age, severity, body weight and other conditions of human patients, but daily dosage for adults is within a range of from about 20 mg to 2000 mg (preferably 20 to 100 mg) which may be given in two to four divided doses. Higher doses may be favorably employed as required.

In addition, the present invention is directed to a method of inhibiting the enzyme squalene synthase which comprises the administration to a subject in need of such treatment a nontoxic therapeutically effective amount of a compound represented by structural formula (I) and pharmaceutically acceptable salts thereof. Specifically, the compounds of this invention are useful as antihypercholesterolemic agents for the treatment of arteriosclerosis, hyperlipidemia, familial hypercholesterolemia and the like diseases in humans. They may be administered orally or parenterally in the form of a capsule, a tablet, an injectable preparation or the like. It is usually desirable to use the oral route. Doses may be varied, depending on the age, severity, body weight and other conditions of human patients, but daily dosage for adults is within a range of from about 20 mg to 2000 mg (preferably 20 to 100 mg) which may be given in two to four divided doses. Higher doses may be favorably employed as required.

The pharmaceutically acceptable salts of the compounds of this invention include those formed from cations such as sodium, potassium, aluminum, calcium, lithium, magnesium, zinc, and from bases such as ammonia, ethylenediamine, N-methyl-glutamine, lysine, arginine, ornithine, choline, N,N'-dibenzylethylenediamine, chloroprocaine, diethanolamine, procaine, N-benzylphenethylamine, diethylamine, piperazine, tris(hydroxymethyl)aminomethane, and tetramethylammonium hydroxide. The salts included herein encompass those wherein one, two or all three of the carboxyl groups are in the salt form. These salts may be prepared by standard procedures.

The compounds of this invention may also be administered in combination with other cholesterol-lowering agents such as those which inhibit another enzyme in the biosynthetic pathway in the synthesis of

cholesterol. Examples of such agents would include but are not limited to HMG-CoA reductase inhibitors, HMG-CoA synthase inhibitors, and squalene epoxidase inhibitors. Illustrative of such HMG-CoA reductase inhibitors are lovastatin, simvastatin, pravastatin and fluvastatin. Examples of HMG-CoA synthase inhibitors are the beta-lactone derivatives disclosed in U.S. Patents 4,806,564; 4,816,477; 4,847,271; and 4,751,237; the beta-lactam derivatives disclosed in U.S. 4,983,597 and U.S.S.N. 07/540,992 filed June 20, 1990; and the substituted oxacyclopropane analogues disclosed in European Patent Publication EP 0 411 703. Illustrative examples of squalene epoxidase inhibitors are disclosed in European Patent Publication EP 0 318 860 and in Japanese Patent Publication J02 169-571A. LDL-receptor gene inducer molecules are disclosed in U.S. Patent Application S.N. 07/670,640 filed March 18, 1991. Other cholesterol lowering agents that may be administered include niacin, probucol, the fibric acids: clofibrate and gemfibrozil, and LDL-receptor gene inducers. Representative of such combinations are those containing about 10-400 mg of a compound of formula (I) in combination with about 20-100 mg of an HMG-CoA reductase inhibitor, 20 to 200 mg of a HMG-CoA synthase inhibitor, or 1 to 200 mg of a squalene epoxidase inhibitor, or 250-1000 mg of probucol, or 600-1200 mg of gemfibrozil, or 1-2 g of clofibrate, or 3-6 g of niacin, or 20-300 mg of an LDL-receptor gene inducer.

The compounds of this invention may also be co-administered with pharmaceutically acceptable non-toxic cationic polymers capable of binding bile acids in a non-resorbable form in the gastro-intestinal tract. Examples of such polymers include cholestyramine, colestipol and poly[methyl-(3-trimethyl)aminopropyl]-imino-trimethylene dihalide. The relative amounts for co-administration of the compounds of this invention and these polymers is between 1:100 and 1:15,000 (w/w).

The intrinsic squalene synthase inhibitory activity of representative compounds of this invention was measured by the standard in vitro protocol described below:

PREPARATION OF HUMAN HepG2 cell ENZYME

1. SOURCE: HEPG2 CELL LINE (Liver, hepatoblastoma, Human) ATCC No. HB 8065

2. CELL GROWTH AND MAINTENANCE

Culture Medium: Minimum essential medium (MEM) with non-essential amino acids, sodium pyruvate, and 10% fetal bovine serum. The medium was changed twice weekly. A confluent monolayer was achieved in 1 week. The growth medium was prepared as listed below.

| Solution | Volume (ml) |
|---|---|
| 1. MEM (Gibco #320-1090AK) With Earle's salts and L-glutamine | 1000 |
| 2. Penicillin (10,000 units/mL), streptomycin (10,000 mg/mL), Gibco #600-5140 PG | 10 |
| 3. MEM sodium pyruvate, 10 mM (100X) Gibco #320-1140 | 10 |
| 4. MEM nonessential amino acids, 10 mM (100X) Gibco #320-1140AG | 10 |
| 5. L-glutamine, 200 mM (100X), Gibco #320-5030AG | 10 |
| 6. Hyclone fetal bovine serum, defined, Hyclone #A-111-L | 100 |

SUBCULTURE PROCEDURE: The medium was removed and washed with PBS (Phosphate-Buffered Saline 15.6 mM, pH 7.0). Fresh trypsin (0.25%)-EDTA (0.02%) with Hank's Balanced Salt solution was added and the flask was allowed to stand for a minute before the trypsin solution was removed. The flask was incubated at 37° C until cells detached. Fresh medium was added and the cells were dispersed and dispensed into new flasks. Subcultivation ratio: 1:6.

PREPARATION OF DELIPIDATED SERUM: Fetal calf serum (100 ml) and CAB-O-Sil (2 grams) were stirred overnight at 4° C and centrifuged at 16,000 rpm for 5 hrs. The supernantant was filtered and the serum was stored at 4° C.

48 hrs. prior to harvest, cells grown in MEM with 10% Fetal Calf serum were switched to MEM with 10% delipidated serum.

HARVEST: The medium was removed and the cells were washed with PBS. Fresh trypsin (0.25%)-EDTA (0.02%) with Hanks' Balanced Salt solution was added and allowed to stand and removed. The flask was incubated at 37° C until the cells detached. MEM medium (6 mL/flask) was added to suspend cells and combined into a centrifuge tube. The cells were spun at 1,000 rpm for 5 mins. The cell pellet was resuspended in PBS and recentrifuged. Cells were counted (2.5 x $10^9$ yield from 18 flasks (75 $cm^2$)), and resuspended in 10 mL of 50mM HEPES (N-[2-Hydroxyethyl]piperazine-N'-[2-ethane-sulfonic acid]) contain-

ing 5mM $MgCl_2$, 2mM $MnCl_2$, 10mM DTT, pH 7.5 (enzyme suspension buffer).

CELL EXTRACTS: The cell suspension was sonicated (probe sonicator setting #60, pulse) on ice for 2 min. After a 1 min. cooling on ice, the sonication was repeated until greater than 90% of the cells were broken as observed microscopically. The supernatant was centrifuged for 10 mins. at 10,000 rpm and the supernantant was transferred to a clean tube and centrifuged at 20,000 rpm for 20 mins. The HepG2 enzyme preparation was centrifuged at 34,000 rpm to separate the cytosol and microsomal enzymes. The resulting pellet from the 34,000 rpm centrifugation, containing the squalene synthase, was resuspended in 5 mL of enzyme suspension buffer. The enzyme suspension was diluted and used to perform the squalene synthase assay using 3$\mu$M $^3$H-farnesyl pyrophosphate as the substrate.

Preparation of Yeast Enzyme

S. cerevisiae W303-1A (MATa ade2-1 can1-100 his3-11,15 leu2-3,112 trp1-1 ura3-1) was grown at 30°C for 24 hours in YPD medium (2% yeast extract, 1% bacto-peptone, 2% glucose) supplemented with 20 $\mu$g/mL adenine. The cells were washed and resuspended in a minimal volume of breakage buffer (100 mM $KPO_4$, pH 7.4, 3 mM DTT, 1 mM PMSF). The cells were added to the small chamber of the Bead-Beater Cell Disrupter (Biospec Products, Bartlesville, OK) containing 100 g of 0.5 mm glass beads. Buffer was added to fill the chamber. The sealed chamber was packed in ice and processed for 4 cycles of 30 sec with 2 min. cooling between cycles. Cell breakage was determined to be >90% by phase-contrast microscopy. The glass beads were removed by Whatman filter paper and the filtrate collected into an ice-immersed filter flask. The extract was centrifuged at 1500 x g for 10 min at 4°C. The pellet (P1) containing unbroken cells and cell walls was discarded. The supernatant (S1) was centrifuged at 30000 x g for 30 min at 4°C. The pellet (P2) contained about two-thirds of the total squalene synthase activity. The supernatant (S2) can be centrifuged at 100,000 x g for 60 min at 4°C to pellet the remaining activity (P3). The pellet fractions were suspended at 10-20 mg/ml protein in breakage buffer containing 25% glycerol. The activity was stable for several months at -80°C.

Squalene Synthase Assay

Reactions were performed in 1.2 mL polypropylene tube strips of eight. The standard reaction mixture in a total volume of 0.140 mL, contained HEPES-Na+ buffer pH 7.5 50 mM, NADPH 1 mM, $MgCl_2$ 5.5 mM, KF 11 mM, DTT 3 mM, terbinafine (Yeast squalene epoxidase inhibitor, Sandoz SF86-327) or mammalian squalene epoxidase inhibitor (Banyu FW-439H) 1 $\mu$g/mL, farnesyl pyrophosphate (FPP) 5$\mu$M (NEN), $^3$H-farnesyl pyrophosphate 0.25 $\mu$Ci (NEN, 20 Ci/mmole), enzyme preparation 0.1 $\mu$g yeast protein or 0.4 $\mu$g HepG2 protein and test sample in 5$\mu$l DMSO. All assays components except FPP/$^3$H-FPP were pre-incubated for 12 min at 30°C to allow inhibitor to bind to the enzyme. The reactions were started with FPP/$^3$H-FPP addition. After 12 min at 30°C, the reactions were stopped by addition of 0.2 mL ethanol. The reaction product was extracted with 0.4 mL heptane containing 0.4 $\mu$L carrier squalene 0.2 mL of the heptane extract was counted by liquid scintillation. The $IC_{50}$ was determined as the concentration of inhibitor which gave 50% of the control (no inhibitor) enzyme activity as determined from a logarithmic curve fit of the data from a drug titration.

Percent inhibition is calculated by the formula:

$$\frac{(\text{Control} - \text{Sample}) \text{ X } 100}{\text{Control} - \text{Blank}}$$

$IC_{50}$ values were determined by plotting the log of the concentration of the test compound versus the percentage inhibition. The $IC_{50}$ is the concentration of inhibitor that gives 50% inhibition as determined from these plots.

Below are $IC_{50}$'s representative of the inherent squalene synthase inhibitory activity of the compound of the present invention.

| Squalene Synthase Inhibition IC50 (μg/mL) | | |
|---|---|---|
| Compound | Yeast Enzyme | HepG2 Enzyme |
| Compound A | 7.5 | 41.6 |
| Compound C | 1 | 19.3 |
| Compound D | 0.22 | 0.29 |

Alternatively, the intrinsic squalene synthase inhibitory activity of the compounds formed in the fermentation of the microorganisms of the present invention may be measured by the standard in vitro protocol described below:

Preparation of Rat Liver Microsomes:

Male, CHARLES RIVER® CD rats (120 to 150 g) were fed a diet containing 0.1% lovastatin for 4 days. The livers from these rats were homogenized in 5 volumes (mL/g) of ice cold 50 mM HEPES (4-(2-hydroxyethyl)-1-piperazine-ethanesulfonic acid), 5 mM EDTA(ethylenediaminetetraacetic acid) pH 7.5 with a Potter-Elvehjem type tissue grinder. The homogenate was centrifuged twice at 20,000 x g for 15 min. at 4°C, discarding the pellet each time. The supernatant was then centrifuged at 100,000 x g for 1 hr at 4°C. The resulting microsomal pellet was resuspended in a volume of the above homogenizing buffer equal to one-fifth the volume of the original homogenate. This microsomal preparation has a protein concentration of about 7 mg/mL. The microsomal suspensions were stored in aliquots at -70°C. Squalene synthase activity in these aliquots is stable for a least several months.

Partial Purification of Prenyl Transferase

Prenyl transferase was purified to use in the enzymatic synthesis of radiolabelled farnesyl pyrophosphate. Prenyl transferase was assayed by the method of Rilling (Methods in Enzymology 110, 125-129 (1985)) and a unit of activity is defined as the amount of enzyme that will produce 1 μmole of farnesyl pyrophosphate per minute at 30°C in the standard assay.

The livers of 23 forty-day old male rats that had been fed 5% cholestyramine plus 0.1% lovastatin were homogenized in a Waring blender in 1 liter of 10 mM mercaptoethanol, 2 mM EDTA, 25 mM leupeptin, 0.005% phenylmethylsulfonyl fluoride, pH 7.0 containing 0.1 trypsin inhibitor units of aprotinin/mL. The homogenate was centrifuged at 20,000 x g for 20 min. The supernatant was adjusted to pH 5.5. with 6 N HOAc and centrifuged at 100,000 x g for 1 hour. This supernatant was adjusted to pH 7.0 with 3 N KOH and a 35-60% ammonium sulfate fraction taken. The 60% pellet was redissolved in 60 mL of 10 mM potassium phosphate, 10 mM mercaptoethanol, 1 mM EDTA pH 7.0 (Buffer A) and dialyzed against two 1 liter changes of Buffer A. This dialyzed fraction was applied to a 12.5 x 5 cm column of DEAE-sepharose 4B equilibrated with Buffer A. The column was washed with 700 mL of Buffer A and a 1 liter gradient from Buffer A to 100 mM potassium phosphate, 10 mM mercaptoethanol, 1 mM EDTA, pH 7.0. Fractions having a specific activity greater than 0.20 units/mg were combined, solid ammonium sulfate was added to bring to 60% saturation and pelleted. The pellet was dissolved in 8 ml of 10 mM Tris, 10 mM β-mercaptoethanol pH 7.0 (Buffer B). The redissolved pellet was taken to 60% saturation with ammonium sulfate by adding 1.5 volumes of saturated ammonium sulfate in Buffer B. This ammonium sulfate suspension contained 3.5 units/mL with specific activity of 0.23 units/mg and was free of isopentenyl pyrophosphate isomerase activity. This ammonium sulfate suspension was used for the synthesis of [4-$^{14}$C]farnesyl-pyrophosphate and its activity was stable stored at 4°C for a least 6 months.

Enzymatic Synthesis of [4-$^{14}$C]farnesyl-pyrophosphate

The solvent (ethanol: 0.15 N $NH_4OH$, 1:1) was removed from 55 mCi of [4-$^{14}$C]isopentenyl pyrophosphate(47.9 mCi/mmole) by rotary evaporation. Six hundred microliters of 100 mM Tris, 10 mM $MgCl_2$, 4 mM dithiothreitol pH 7.5 was added and the solution was transferred to a 1.5 mL Eppendorf centrifuge tube. Geranyl-pyrophosphate, 250 mL of a 20 mM solution, and 50 mL of the ammonium sulfate suspension of prenyl transferase were added to initiate the reaction. This incubation contained 5 mmoles of geranyl pyrophosphate, 1.15 mmoles of isopentenyl pyrophosphate, 6 mmoles of $MgCl_2$ of 0.18 units of prenyl transferase in a volume of 900 mL. The incubation was conducted at 37°C. During the incubation, the mix turned cloudy white as the newly formed magnesium complex of farnesyl pyrophosphate precipitated out of solution. The [4-$^{14}$C]farnesyl pyrophosphate was collected by centrifugation for 3 minutes at 14,000 rpm in an Eppendorf centrifuge tube, the supernatant removed, and the pellet was dissolved in 1.0 mL of 50 mM HEPES, 5 mM EDTA, pH 7.5. The yield was 50.7 mCi (92%) of [4-$^{14}$C]farnesyl pyrophosphate. The [4-$^{14}$C]farnesyl pyrophosphate was stored in aliquots at -70°C.

Squalene Synthase Assay

Reactions were performed in 16 x 125 mm screw cap test tubes. A batch assay mix was prepared from the following solution:

| | | mL per assay | volume for 50 assays |
|---|---|---|---|
| 1. | 250 mM HEPES pH 7.5 | 20 | 1000 |
| 2. | NaF 110 mM | 10 | 500 |
| 3. | $MgCl_2$ 55 mM | 10 | 500 |
| 4. | Dithiothreitol 30 mM | 10 | 500 |
| 5. | NADPH 10 mM (made fresh) | 10 | 500 |
| 6. | [4-$^{14}$C]farnesyl-pyrophosphate 47.9 mCi/mmole, and 0.025 mCi/3.0 mL | 3.0 | 150 |
| 7. | $H_2O$ | 24 | 1200 |

This assay mix was degassed under a vacuum and flushed with $N_2$. Solutions of the squalene synthase inhibitors were prepared either in DMSO or MeOH and a 1:120 dilution of the microsomal protein was made with the original homogenizing buffer. For each reaction, 87 mL of the assay mix was taken with 3 mL of an inhibitor solution DMSO or MeOH in the controls), warmed to 30°C in a water bath and then the reaction was initiated by the addition of 10 mL of the 1:120 dilution of microsomal protein (0.6 mg protein total in the assay). The reactions were stopped after 20 minutes by the addition of 100 mL of a 1:1 mix of 40% KOH with 95% EtOH. The stopped mix was heated at 65°C for 30 min., and cooled. Ten mL of heptane was added and the mix was vortexed. Two g of activated alumina was then added, the mix vortexed again, the alumina allowed to settle and 5 mL of the heptane layer was removed. Ten mL of scintillation fluid was added to the heptane solution and radioactivity was determined by liquid scintillation counting.

Percent inhibition is calculated by the formula:

$$1 - \frac{[\text{Sample} - \text{Blank}]}{[\text{Control} - \text{Blank}]} \times 100$$

Representative of the squalene synthetase inhibitory character of the compounds of this invention is the datum below.

| Compound | Squalene Synthase $IC_{50}$ |
|---|---|
| Compound A | 15 µM |

The present compounds also demonstrate broad spectrum antifungal activity as determined by broth dilution methods. The compounds are particularly active towards filamentous fungi and yeasts including Candida albicans and Ustilago zeae. The sensitivity of filamentous fungi and yeast was determined using inhibitor dilution assays in microtiter format. The compounds were dissolved in DMSO at 2 mg/mL and diluted in 100 µL of DMSO. Exponential phase Candida, Cryptococcus and Ustilago cells were diluted in fresh YNB/G (Difco Yeast Nitrogen Base supplemented with 2% glucose) and YCB/Glu (Difco yeast carbon base with 2 mM 1-glutamic acid) such that the inoculum was 1 x $10^4$ cells/mL. Aspergillus spores were harvested from a well-sporulated Sabouraud Dextrose Agar slant in 0.4% Tween 80 and diluted into media to give an inoculum of 1 x $10^3$ spores/mL. The wells were filled with 150 µL of inoculated media. The final drug concentration tested ranged from 40 to 0.313 µg/mL. The microtiter dishes were incubated at 29°C for 20 to 48 hours. The minimum inhibitory concentration (MIC) is defined as the lowest concentration to prevent visible growth after incubation for 20 hours at 29°C for the yeasts and 24 to 48 hours at 29°C for

the filamentous fungi. Representative of the antifungal activity are the minimum inhibitory concentration data shown below.

| Minimum Inhibitory Concentration (mg/mL) | | | | |
|---|---|---|---|---|
| Organism | Medium | COMPOUND | | |
| | | A | C | D |
| Candida albicans MY1055 | YNB/G<br>YCB/Glu | 40<br>5 | >40<br>2.5 | >40<br>20 |
| Cryptococcus neoformans MY2061 | YNB/G<br>YCB/Glu | 40<br>1.25 | >40<br>1.25 | 40<br>10 |
| Cryptococcus neoformans MY2062 | YNB/G<br>YCB/Glu | >40<br>2.5 | >40<br>5 | >40<br>10 |
| Ustilago zeae MF1996 | YNB/G<br>YCB/Glu | 5<br>1.25 | 10<br>2.5 | 20<br>2.5 |
| Aspergillus fumigatus MF4839 | YNB/G<br>YNB/Glu | >40<br>0.625 | >40<br>0.625 | >40<br>1.25 |

Thus the present invention is also directed to a method of inhibiting fungal growth which comprises the application to the area in which growth is to be controlled an antifungally effective amount of a compound of Formula (I). Additionally, the present invention is directed to a method of treating fungal infections which comprises the administration to an organism in need of such treatment a nontoxic therapeutically effective amount of a compound represented by the structural formula (I) and pharmaceutically acceptable salts thereof. Based on the above MIC data it is determined that generally from 2 to about 20 mg/kg should be employed as a unit dosage in an antifungal treatment.

The compounds of this invention are adaptable to being utilized in various applications of antifungal compositions. In such use, compounds may be admixed with a biologically inert carrier, generally with the aid of a surface active dispersing agent, the nature of which would vary depending on whether the use is for the control of pathogens infecting mammals such as man, or birds or reptiles, or for control of fungi in agriculture such as in soil or plant parts, or for the control of fungi in inanimate objects.

In compositions for medical applications, the compounds may be admixed with a pharmaceutically acceptable carrier, the nature of which will vary depending on whether the composition is to be topical, parenteral or oral.

If said application is to be topical, the drug may be formulated in conventional creams and ointments such as white petroleum, anhydrous lanolin, cetyl alcohol, cold cream, glyceryl monostearate, rose water and the like.

For parenteral applications, the compounds may be formulated in conventional parenteral solutions such as 0.85 percent sodium chloride or 5 percent dextrose in water, or other pharmaceutically acceptable compositions.

Compositions for oral administration may be prepared by intimately mixing the component drugs with any of the usual pharmaceutical media, including, for liquid preparations, liquid carriers such as water, glycols, oils, alcohols, and the like; and for solid preparations such as capsules and tablets, solid carriers such as starches, sugars, kaolin, ethyl cellulose, surface active dispersing agents, generally with lubricant such as calcium stearate, together with binders, disintegrating agents and the like.

These compositions are then administered in amounts sufficient to obtain the desired antifungal effect. For medical application, the method comprises administering to a subject in need of treatment a therapeutically effective antifungal amount of a compound of Formula I. The appropriate doses will vary depending on age, severity, body weight and other conditions. For topical application the compositions are applied directly to the area where control is desired. For internal administration, the composition may be applied by injection or may be administered orally.

For non-medical application, the product of the present invention, either singly or as a mixture, may be employed in compositions in an inert-carrier which includes finely divided dry or liquid diluents, extenders, fillers, conditioners and excipients, including various clays, diatomaceous earth, talc, and the like, or water and various organic liquids such as lower alkanols, for example ethanol and isopropanol, or kerosene, benzene, toluene and other petroleum distillate fractions or mixtures thereof.

These compositions may be employed by applying to the surface of or incorporating in the medium to be protected. For the control of rice blast, tomato late blight, tomato early blight, wheat leaf rust, bean powdery mildew and tomato Fusarium wilt, the compositions may be applied directly to the plant in topical application or administered to the soil for systemic application. The method comprises administering to the affected plant, soil or medium to be protected an antifungally effective amount of the compound of Formula I.

The compounds of Formula I also exhibit Farnesyl-transferase inhibition, as shown below:

FARNESYL-TRANSFERASE ASSAY

I. PREPARATION OF SOLUTIONS REQUIRED FOR THE ASSAY

i. 1.0 M $MgCl_2$ (MW = 203.3)

203.3 g $MgCl_2$ was dissolved in 1.0 L distilled water, and filtered through a sterile filter and stored at 4°C.

ii. 1.1 M HEPES (MW = 238.3) and 55.5 mM $MgCl_2$

264.5 g of HEPES was added to 700 mL distilled water followed by 56 mL of 1.0 M $MgCl_2$. The pH was adjusted to 7.5 with sodium hydroxide and the volume was brought to 1000 mL. The solution was filtered through a sterile filter and stored at 4°C.

iii. 0.5 M Dithiothreitol (DTT. MW = 154.24)

77.12 mg of DTT was dissolved in 1.0 mL of distilled water. A few drops of 10 N NaOH was added to get the DTT to go into solution. 0.5 M DTT was divided into 500 µL aliquots and stored at -20°C.

iv. Preparation of Assay Buffer (Prepare fresh daily)

100 µL of 0.5 M DTT was added to 900 µL of 1.1 M HEPES, pH 7.5 and 55.5 mM $MgCl_2$ to yield a 10X buffer consisting of 1.0 M HEPES, pH 7.5, 50 mM $MgCl_2$ and 50 mM DTT.

v. Preparation of unlabelled farnesyl pyrophosphate (FPP)

The unlabelled FPP (MW=433.3) was provided as a 1.8 mM (1mg/1.28 mL) solution. This solution was diluted to 2 µM with water and prepared fresh weekly and stored at -20°C. Glass tubes were used when preparing the unlabelled FPP.

vi. Preparation of $^3$H-FPP

The $^3$H-FPP (NEN #NET-1042, MW=433.3) was used directly from the bottle as provided by NEN. The stock concentration of the $^3$H-FPP was 25 µM.

vii. Preparation of the reaction mix for Totals and unknown samples (on ice)

The reaction mix was prepared as below:

| Reagent | Stock Conc. | Vol to use/assay | Conc. in 20 µL | Final Conc. in assay |
|---|---|---|---|---|
| $^3$H-FPP | 25 µM | 0.2µL | 0.25 µM | 50 nM |
| Cold-FPP | 2000 nM | 11.25µL | 1.125µM | 225 nM |
| ras-CVLS | 1 mg/mL (47.62 µM) | 6.3 µL | 15 µM | 3µM |
| d-$H_2O$ | | 2.25µL | | |
| **TOTAL VOLUME** | | 20 µL | | |

A Blank reaction mix was also prepared with the negative control ras-protein, and the Total/Sample

reaction mix received the ras-CVLS. The same volumes were used to prepare the Blank reaction mix as those in Table 1.

viii. Preparation of the Farnesyl Transferase solution

The farnesyl trasferase solution was prepared as shown below. The farnesyl transferase mix was prepared right before use.

| Reagent | Stock Conc. | Vol to use/assay |
|---|---|---|
| d-$H_2O$ | | 14.0 µL |
| Buffer | stock = 10X | 10.0 µL |
| F.Transferase | stock = 1 mg/mL | 1.0 µL |
| **Total Volume** | | 25 µL |

ix. Preparation of 1.0 M HCl in 100% Ethanol

The stop solution was prepared by mixing 43 mL of concentrated HCl (11.6 M) with 457 mL of 100% ethanol to yield 1.0 M HCl in ethanol.

V. STEPS FOR THE ASSAY

i. The assay volume was 100 µL, the volume of sample to be tested was 5 µL and the time of incubation is 60 min at room temperature.
ii. The TECAN 8000/505, an automatic pipetting station, added 50 µL of water plus 5 µL of sample to the assay tubes.
iii. The ras-CVLS reaction mix (20 µL) was manually added to totals and unknowns. The negative control ras-protein reaction mix (20 µL) was manually added to the blank tubes.
iv. The farnesyl transferase mix (25 µL) was manually added to all tubes to initiate the reaction and the assay tubes were kept at room temperature for 60 min.
v. The assay was stopped placing the assay tubes in an ice bath for 5 minutes.
vi. 1.0 mL of 1.0 M HCl in 100% ethanol was then added to each tube. The assay tubes (uncapped) were incubated at 37°C for 60 min. to facilitate the hydrolysis of the FPP.
vii. Assay tubes were harvested through a TOMTEC 96-well harvestor (6 x 16 with extended tips, or SKATRON cell harvestor) onto LKB double thickness filter mats.
viii. The cell harvestor was adjusted so that each well was washed with 10 mL of 100% ethanol.
ix. Filter mats were baked in microwave oven for 6-8 min. to dry the filtermats.
x. The filter mats were placed in counting bags, 30 mL of LKB β-scint cocktail was added and the filter mats were counted for 120 sec. in the LKB β-plate counter.

VI. RESULTS AND DISCUSSION

Percent inhibition was calculated according to the following equation:

$$\% \text{ Inhibition} = \frac{[\text{Total} - \text{Sample}] \times 100}{[\text{Total} - \text{Blank}]}$$

The Farnesyl Transferase data presented below is a measurement of the ability of the test compound to inhibit Ras farnesylation in vitro.

| Compound | $IC_{50}$ (μM) |
|---|---|
| Compound A | 8 |
| Compound C | 4.5 |
| Compound D | 3.3 |

The pharmaceutical compositions containing the compounds of structural formula (I) inhibit farnesyl-protein transferase and the farnesylation of the oncogene protein Ras. These compounds are useful as pharmaceutical agents for mammals, especially for humans. These compounds may be administered to patients for use in the treatment of cancer. Examples of the type of cancer which may be treated with the compounds of this invention include, but are not limited to, colorectal carcinoma, exocrine pancreatic carcinoma, and myeloid leukemias.

The compounds of this invention may be administered to mammals, preferably humans, either alone, or preferably, in combination with pharmaceutically-acceptable carriers or diluents, optionally with known adjuvants, such as alum, in a pharmaceutical composition, according to standard pharmaceutical practice. The compounds can be administered orally or parenterally, including intravenous, intramuscular, intraperitioneal, subcutaneous and topical administration.

For oral use of a chemotherapeutic compound according to this invention, the selected compounds may be administered, for example, in the form of tablets or capsules, or as an aqueous solution or suspension. In the case of tablets for oral use, carriers which are commonly used include lactose and corn starch, and lubricating agents, such as magnesium stearate, are commonly added. For oral administration in capsule form, useful diluents include lactose and dried corn starch. When aqueous suspensions are required for oral use, the active ingredient is combined with emulsifying and suspending agents. If desired, certain sweetening and/or flavoring agents may be added. For intramuscular, intraperitoneal, subcutaneous and intravenous use, sterile solutions of the active ingredient are usually prepared, and the pH of the solutions should be suitably adjusted and buffered. For intravenous use, the total concentration of solutes should be controlled in order to render the preparation isotonic.

The present invention also encompasses a method of the treatment of cancer, comprising the administration of a pharmaceutical composition comprising a therapeutically effective amount of the compounds of this invention, with or without pharmaceutically acceptable carriers or diluents.

Suitable compositions of this invention include aqueous solutions comprising compounds of this invention and pharmacologically acceptable carriers, e.g. saline, at a pH level, e.g., 7.4. The solutions may be introduced into a patient's intramuscular blood-stream by local bolus injection.

When a compound according to this invention is administered into a human subject, the daily dosage will normally be determined by the prescribing physician with the dosage generally varying according to the age, weight, and response of the individual patient, as well as the severity of the patient's symptoms.

In one exemplary application, a suitable amount of compound is administered to a human patient undergoing treatment for cancer. Administration occurs in a amount between about 0.1 mg/kg of body weight to about 20 mg/kg of body weight of a mammal per day, preferably of between 0.5 mg/kg of body weight to about 10 mg/kg of body weight of a mammal per day.

The following examples illustrate the preparation of the compounds of formula (I) and their incorporation into pharmaceutical compositions and, as such, are not to be considered as limiting the invention set forth in the claims appended hereto.

The composition of media employed in the following Examples are listed below:

| KF SEED MEDIUM | per liter |
|---|---|
| Corn Steep Liquor | 5 g |
| Tomato Paste | 40 g |
| Oat Flour | 10 g |
| Cerelose | 10 g |
| Trace Element Mix #2 | 10 ml |
| Distilled Water | 1000 ml |

pH adjusted to 6.8 (presterile)
50 mL/non-baffled 250 mL Erlenmeyer flask
autoclave 20 minutes (121°C, 15 psi)

| Trace Element Mix #2 | g/L |
|---|---|
| $FeSO_4 \bullet 7H_2O$ | 1.0 |
| $MnSO_4 \bullet 4H_2O$ | 1.0 |
| $CuCl_2 \bullet 2H_2O$ | 0.025 |
| $CaCl_2 \bullet 2H_2O$ | 0.1 |
| $H_3BO_3$ | 0.056 |
| $(NH_4)_6Mo_7O_{24} \bullet 4H_2O$ | 0.019 |
| $ZnSO_4 \bullet 7H_2O$ | 0.2 |

dissolved in 1L 0.6 N HCl

Solid Substrate Production Medium

Solid substrate media prepared in nonbaffled 250 mL Erlenmeyer flasks

| BRF | |
|---|---|
| Brown rice | 5.0 g/flask |
| Base liquid #3 | 20.0 mL/flask |

| Base liquid #3 | |
|---|---|
| | g/L |
| Yeast extract | 1.0 |
| Sodium tartrate | 0.5 |
| $KH_2PO_4$ | 0.5 |
| distilled water | 1000.0 mL |
| (no pH adjustment) | |

autoclave 15 minutes (121°C, 15 psi)
add 15.0 mL distilled water per flask
autoclave 20 minutes (121°C, 15 psi)

EXAMPLE 1

Preparation of Compound A

A. Culturing MF5628

Culture MF5628 was inoculated into 3 KF seed medium flasks using a mixture of hyphae and spores preserved in sterile soil. The KF seed flasks were incubated for 74 hours at 25° C, 220 rpm, 85% humidity. At the end of this incubation, culture growth from the 3 seed flasks was pooled and then 2.0 mL aliquots were aseptically transferred to each of 50 BRF solid production medium flasks. These production flasks were then incubated statically at 25° C, 85% humidity for 21 days. At harvest 50 mL methyl ethyl ketone (MEK) was added to each BRF production flask and the solid growth was manually broken apart into smaller pieces. Solvent treated flasks were returned to the gyrotory shaker for agitation at 220 rpm for 30 minutes in order to further break apart the mycelial mass as well as to improve contact of the solvent with the cells. After shaking, the contents of these flasks were pooled by pouring the entire contents of the flasks (solids and all) into a 2L flask.

B. Isolation of Compound A

The 21-day 2000 mL solid fermentation in BRF media from above was extracted with 2500 mL of methyl ethyl ketone (MEK) by stirring an additional 1.5 hours at room temperature. The extracted fermentation was then filtered through CELITE®.

A portion of the MEK extract (300 mL) was concentrated to dryness in vacuo. The resulting residue was partitioned between 50 mL hexanes and 50 mL methanol. The methanol layer was concentrated to dryness in vacuo. The residue was dissolved in 100 mL of 1:1 MeOH:100 mM NaOAc pH 4.8 and again extracted with 50 mL hexanes. The aqueous methanol layer was then loaded at 150 mL/hour onto a column of BioRad AG4-X4(acetate) (vol. = 28ml,25mm x 50 mm, 100 - 200 mesh) which had been equilibrated with 1:1 MeOH:100 mM NaOAc pH 4.8 (equilibration buffer). The column was then washed with 100 mL of equilibration buffer and then with 100 mL of 6:4 acetonitrile ($CH_3CN$):$H_2O$. The column was eluted with 0.16 N sulfuric acid in 6:4 $CH_3CN$:$H_2O$ collecting 8 mL fractions. Fractions 21 to 30 (6.0-8.6 column volumes) contained crude Compound A and were pooled (vol. = 100ml, pH 2.54) and extracted with 100 mL of ethyl acetate. The ethyl acetate layer was concentrated in vacuo to yield 384 mg crude Compound A which was dissolved in 20 mL methanol.

A portion (5.2mL, 100mg) of the crude Compound A in methanol was concentrated to dryness in vacuo and dissolved in 1 mL of HPLC mobile phase. This solution was chromatographed on Whatman Partisil 10 ODS 3 (22 mm x 25 cm) using 45% $CH_3CN$/55% $H_2O$ containing 0.1% $H_3PO_4$ at 20ml/min. The column was monitored at 220 nm and 0.4 min (8 ml) fractions were collected. Compound A eluted in fractions 44-49. Analytical HPLC (Partisil 5 ODS 3, 4.6 mm x 25 cm, 55% $CH_3CN/H_2O$ containing 0.1% $H_3PO_4$, 1.0 mL min, detection at 220nm) showed fractions 44 to 49 to contain essentially pure Compound A, $t_r$ = 4.25'. Fractions 44 to 49 were pooled and extracted with an equal volume (48 ml) of $CH_2Cl_2$, the $CH_2Cl_2$ layer removed, dried over anhydrous $Na_2SO_4$ and concentrated in vacuo to yield 20.6 mg pure Compound A.

EXAMPLE 2

Preparation of Compound C

A. Culturing MF 5628

Culture MF 5628 was grown according to the procedures of Example 1, Step A.

B. Isolation of Compound C

A 1500 mL portion of the MEK extract from Example 1 Part B was processed through BioRad AG4x4 as described for the preparation of Compound A to yield a 1.94 g mixture of crude compounds A, C and D. A portion (0.95 g) of this mixture was separated by high speed countercurrent chromatography using the apparatus manufactured by P.C. Inc. (11805 Kim Place, Potomac, Maryland, USA.) The crude mixture was concentrated in vacuo to dryness and dissolved in 4 ml of the upper phase and 4 ml of the lower phase of a solvent system consisting of 5 parts hexanes: 5 parts ethyl acetate: 5 parts methanol: 5 parts 0.1% aqueous $H_3PO_4$. The sample was applied to the tail of a #10 preparative multilayer coil (P.C. Inc.) which had been filled completely with the lower phase of the above solvent system. The coil was then eluted with the upper phase of the above solvent system from the tail of the column to the head of the column at 3.0 ml/min, at a rotation speed of 800 rpm in the forward direction. Fractions of 7.5 ml were collected and analyzed by RP HPLC as described in Example 1 Part B for the preparation of Compound A. Fractions 75 to 95 contained 95 mg of Compound C.

EXAMPLE 3

Preparation of Compound D

A. Culturing MF 5628

Culture MF 5628 was grown according to the procedures of Example 1, Step A.

B. Isolation of Compound D

The isolation of Compound D was performed as described in Example 2. Fractions 120 to 160 of the high counter current separation contained 30 mg of crude Compound D. One half (15 mg) of this mixture was further purified by chromatography on a column Phenomenex Ultracarb 5 ODS 30, 15 cm X 10 mm, eluted at 4.0 ml/min with a 1:1 mixture of solution A and B. Solution A was 0.1% $H_3PO_4$ in 400 ml acetonitrile and 600 ml water and solution B was 0.1% $H_3PO_4$ in 750 ml acetonitrile and 250 ml water. Fractions of 2 ml each were collected and numbers 24 to 27 were pooled, an equal volume of $H_2O$ added and the solution extracted with an equal volume of EtOAc. The EtOAc layer was concentrated to dryness in vacuo and contained 10.2 mg of Compound D.

EXAMPLE 4

Preparation of an Ammonium Salt

A 0.1 mmol sample of the free acid of a compound of formula (I) is dissolved in 10 mL ethyl acetate. The resulting solution is saturated with gaseous ammonia and the ammonium salt precipitates from solution.

EXAMPLE 5

Preparation of a Potassium Salt

A solution of 0.1 mmol of the free acid of a compound of formula (I) in 10 mL methanol is treated with an aqueous or methanolic solution containing 0.3 mmol of potassium hydroxide. Evaporation of the solvent affords the tri-potassium salt. Addition of between 0.1 and 0.3 mmol of potassium hydroxide yields analogously mixtures of the mono-potassium, di-potassium and tri-potassium salts whose composition depends upon the exact amount of potassium hydroxide added.
In a similar fashion, the sodium and lithium salts can be formed.

EXAMPLE 6

Preparation of a Calcium Salt

A solution of 0.1 mmol of the free acid of a compound of formula (I) in 20 mL 6:4 methanol:water is treated with an aqueous solution of 0.1 mmol of calcium hydroxide. The solvents are evaporated to give the corresponding calcium salt.

EXAMPLE 7

Preparation of an Ethylenediamine Salt

A solution of 0.1 mmol of the free acid of a compound of formula (I) in 10 mL of methanol is treated with 0.1 mmol of ethylenediamine. Evaporation of the solvent affords the ethylenediamine salt.
The procedure can also be applied to the preparation of the N,N''-dibenzylethylenediamine salt.

EXAMPLE 8

Preparation of a Tris(hydroxymethyl)aminomethane Salt

To a solution of 0.1 mmol of the free acid of a compound of formula (I) in 10 mL of methanol is added

from 0.1 to 0.3 mmol of tris(hydroxymethyl)aminomethane dissolved in 10 mL methanol. Evaporation of the solvent gives a corresponding salt form, the exact composition of which is determined by the molar ratio of amine added. Similarly prepared are the salts of L-ornithine, L-lysine, and N-methylgluatamine.

EXAMPLE 9

Preparation of an L-arginine Salt

A solution of 0.1 mmol of the free acid of a compound of formula (I) in 20 mL of 6:4 methanol: water is treated with an aqueous solution of 0.1 to 0.3 mmol of L-arginine. Evaporation of the solvent affords the title salt, the exact composition of which is determined by the molar ratio of amino acid to the free acid of formula (I) used.
Similarly prepared are the salts of L-ornithine, L-lysine and N-methylglutamine.

EXAMPLE 10

Preparation of a Compound B (Method 1)

Compound A (0.6 mg) was dissolved in 1 mL diethyl ether and stirred at 0 ° C. Etheral cyanamide was added dropwise until the solution remained yellow. The solution was evaporated under a stream of nitrogen to yield Compound B.

Starting with Compounds C and D, the corresponding trimethyl esters may be prepared, following the procedures above.

EXAMPLE 11

Preparation of a Compound B (Method 2)

To 5 mg of Compound A in methanol (5 mL) was added 2 mL of freshly distilled diazomethane in ether (2.05 M). After 5 minutes the solvent is removed to afford trimethyl ester (Compound B) as an oil.

Starting with Compounds C and D, the corresponding trimethyl esters may be prepared following the above procedure.

EXAMPLE 12

Preparation of Compound B (Method 3)

A solution of 5 mg of Compound A in 0.5 mL of tetrahydrofuran (THF) is treated at room temperature with 3 equivalents of N,N'-diisopropyl-O-benzyl isourea for 18 hours. The reaction mixture is then chilled to -15 ° C, filtered to remove the urea. The filtrate is concentrated under reduced pressure to yield Compound B.
The method of Example 12 is also suitable for the preparation of other ester derivatives such as: 1) methyl and the other lower alkyls, and 2) substituted benzyl esters, using the appropriately substituted isourea. By varying the number of equivalents of the substituted isourea used, the mono-, di- and tri-substituted esters may be selectively prepared. The method of Example 10 is also suitable for the preparation of mono; di and tri-substituted esters of Compounds C and D.

Mass Spectral Data

Mass spectra were recorded on Finnigan-MAT model MAT212 (Electron Impact, EI, 90 eV) and TSQ70B (Fast Atom Bombardment, FAB, EI 70eV) mass spectrometers. Exact mass measurements were performed at high resolution (HR-EI) using perfluorokerosene (PFK) as internal standard. FAB mode used as matrices 5:1 dithiothreitol/dithio-erythritol (DTT/DTE, negative ion mode) DTT/DTE doped with lithium acetate, and thioglycerol doped with acetic acid (positive ion mode).

$^{13}C$ NMR Data

$^{13}C$ NMR spectra were recorded in $CD_3OD$ at 75 MHz on a Varian XL-300 spectrometer. Chemical

shifts are given in ppm relative to the solvent peak at 49.0 ppm ($CD_3OD$) as internal standard.

$^1$H NMR Spectra

$^1$H NMR spectra were recorded at 300 MHz on a Varian XL-300 spectrometer. Chemical shifts are shown in ppm relative to the solvent peaks at 3.30 ppm ($CD_3OD$)as internal standards.

Physical Properties of the compounds of Structure I:

Compound A-the compound of structure (I) wherein R is p-hydroxybenzyl and $Z_1$, $Z_2$ and $Z_3$ are each hydrogen.

Mass Spectral Data:

This compound has the molecular weight 591 by FAB MS (observed $[M-H]^-$ at m/z 590 and $[M+H]^+$ at m/z 592). The molecular formula was determined by exact mass measurement of the trimethyl ester (calc. for $C_{33}H_{45}NO_8$ 583.3145 M-($CH_3OH$ + $H_2O$), found 583.3131).

$^1$H nmr (300 MHz, 18 mg in 0.7 ml $CD_3OD$):$\delta$ 0.90 (t,7,3H), 1.29 (m, 14H), 1.53 (m, 4H), 1.98 (m, 2H), 2.44 (t, 7.2, 4H), 2.62 (d, 16.3, 1H), 2.89 (dd, 8.7, 13.3, 1H), 2.91 (d, 16.3, 1H), 3.11 (dd, 5.3, 14.3, 1H), 3.22 (d, 8.5, 1H), 4.61 (dd, 5.0, 8.7, 1H), 5.5 (m, 2H), 6.68 (d, 8.8, 2H), 7.03 (d, 8.8, 2H).

$^{13}$C nmr (75 MHz, 18 mg in 0.7 ml $CD_3OD$):$\delta$ 14.42, 23.68, 24.83, 24.91, 29.83, 29.88, 30.06, 30.26, 30.29, 32.89, 33.46, 37.50, 43.10, 43.49 (2), 55.08, 57.77, 77.93, 116.21 (2), 124.52, 128.71, 131.38 (2), 137.56, 157.38, 173.55, 173.83, 174.48, 175.75, 214.62.

UV - (100 $\mu$g/ml in $CH_3OH$): 225 nm (8050), 277 nm (1420).

Compound B-the trimethyl ester of Compound A, i.e., the compound of structure (I) wherein R is p-hydroxybenzyl and $Z_1$, $Z_2$ and $Z_3$ are each methyl.

Mass Spectral Data:

This compound has the molecular weight 633 by FAB-MS (observed $[M+Li]^+$ at m/z 640.)

$^1$H nmr (300 MHz, 0.6 mg in 0.7 ml $CD_3OD$):$\delta$ 0.901 (t, 7.6) 1.295 (m), 1.536 (m), 1.992 (M), 2.439 (t, 7.3), 2.588 (d, 16.3), 2.865 (dd, 9.6, 14.3), 2.932 (d, 16.2), 3.073 (dd, 5.4, 14.2), 3.220 (d, 8.1), 3.641 (s), 3.709 (s), 3.716 (s), 4.610 (dd, 5.5, 8.7), 5.515 (m), 6.671 (d, 9.0), 6.988 (d, 8.1).

Compound C-the compound of structure (I) wherein R is benzyl and $Z_1$, $Z_2$ and $Z_3$ are each hydrogen.

Mass Spectral Data:

This compound has the molecular weight of 575 by FAB-MS.

$^1$H nmr (300 MHz 6.2 mg in 0.5 ml $CD_3OD$):$\delta$ 0.90 (t, 7, 3H), 1.29 (brm, 14H), 1.53 (m, 4H), 1.97 (m, 2H), 2.44 (t, 6.6, 4H), 2.56 (d, 16, 1H), 2.88 (d, 16, 1H), 2.98 (dd, 9.4, 14.3, 1H), 3.24 (5, 14.3 1H), 4.69 (dd, 4.9, 9.4, 1H), 5.54 (m, 2H), 7.24 (m, 5H).

$^{13}$C nmr (75 MHz, 6.2 mg in 0.5 ml $CD_3OD$):$\delta$ 14.41, 23.67, 24.81, 24.92, 29.84, 29.88, 30.08, 30.25, 30.29, 32.89, 33.46, 38.24, 42.98, 43.48 (2), 54.81, 57.90, 77.90, 124.49, 127.88, 129.45 (2), 130.38 (2), 137.50, 138.18, 173.49, 173.79, 174.28, 175.63, 214.50.

Compound D- the compound of structure (I) wherein R is -$CH_2$-3-indolyl and $Z_1$, $Z_2$ and $Z_3$ are each hydrogen.

Mass Spectral Data:

This compound has the molecular weight of 614 by FAB-MS.

$^1$H nmr (300 MHz, 10.2 mg in 0.7 ml $CD_3OD$):$\delta$ 0.90 (t, 6.3, 3H), 1.29 (m, 14H), 1.51 (m, 4H), 1.94 (m, 2H), 2.40 (t, 7.4, 2H), 2.42 (t, 7.4, 2H), 2.60 (d, 16.0, 1H), 2.90 (d, 16.0, 1H), 3.21 (d, 6.7, 1H), 3.22 (dd, 6.7, 14.5, 1H), 3.35 (dd, 4.9, 14.5, 1H), 4.76 (dd, 4.9, 8.0, 1H), 5.53 (m, 2H), 6.97-7.11 (m, 2H), 7.10 (s, 1H), 7.31 (brd, 8.0, 1H), 7.56 (brd, 7.6, 1H)

$^{13}$C nmr (75 MHz, 10.2 mg in 0.7 ml $CD_3OD$), $\delta$ 14.42, 23.68, 24.79, 24.90, 28.38, 29.76, 30.02, 30.25, 30.28, 32.89, 33.39, 43.06, 43.46, (2), 54.33, 57.74, 77.98, 110.63, 112.29, 119.33, 119.80, 122.39, 124.46, 124.61, 128.72, 137.68, 138.01, 139.49, 173.49, 173.85, 174.85, 175.67, 214.59

UV (100 $\mu$g/mL in MeOH): 221 nm, 274 nm, 281 nm and 290 nm.

## Claims

**1.** A compound of structural formula (I)

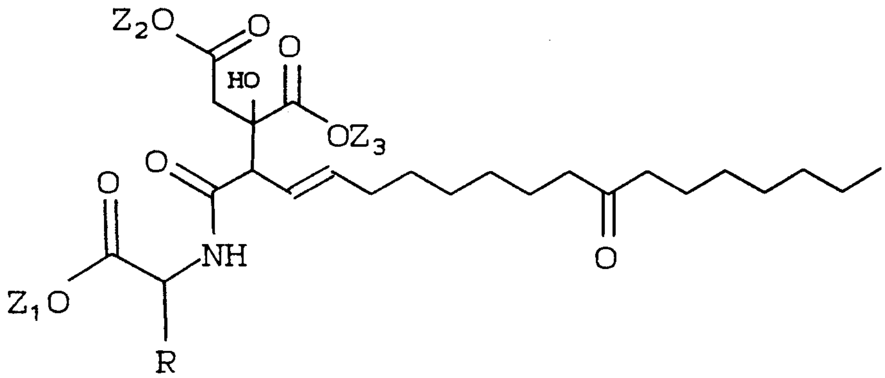

wherein
R is

a) 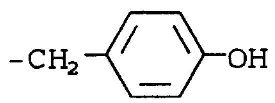 ,

b) 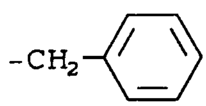 , or

c) 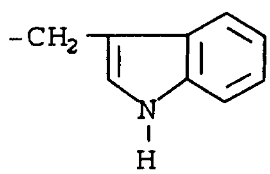 ; and

$Z_1$, $Z_2$ and $Z_3$ are each independently
- a) H;
- b) $C_{1-5}$ alkyl; or
- c) $C_{1-5}$ alkyl substituted with
  - i) phenyl, or
  - ii) phenyl substituted with methyl, methoxy, Cl, Br, I, F or hydroxy;

or a pharmaceutically acceptable salt of a compound of formula (I).

**2.** The compound of Claim 1 in which $Z_1$, $Z_2$ and $Z_3$ are each hydrogen or a pharmaceutically acceptable mono, di or tri salt thereof.

**3.** The compound of Claim 1 in which $Z_1$, $Z_2$ and $Z_3$ are each methyl.

**4.** A pharmaceutical composition comprising a nontoxic therapeutically effective amount of a compound of Claim 1 and a pharmaceutically acceptable carrier.

**5.** A pharmaceutical composition comprising a nontoxic therapeutically effective amount of a compound of

Claim 1 in combination with a pharmaceutically acceptable non-toxic cationic polymer capable of binding bile acids in a non-resorbable form in the gastrointestinal tract and a pharmaceutically acceptable carrier.

**6.** A pharmaceutical composition comprising a nontoxic therapeutically effective amount of a compound of Claim 1 in combination with a nontoxic therapeutically effective amount of a cholesterol lowering agent selected from the group consisting of:

a) HMG-CoA reductase inhibitor;
b) HMG-CoA synthase inhibitor;
c) squalene epoxidase inhibitor;
d) probucol;
e) niacin;
f) gemfibrozil;
g) clofibrate; and
h) LDL-receptor gene inducer.

**7.** The use of a compound as claimed in claim 1 for the manufacture of a medicament for the treatment of hypercholesterolemia.

**8.** A method for inhibiting fungal growth comprising applying to the area where growth is to be controlled an antifungally effective amount of a compound of Claim 1.

**9.** The use of a compound as claimed in claim 1 for the manufacture of a medicament for inhibiting farnesyl-protein transferase and farnesylation of the oncogene protein Ras.

**10.** A process of making a compound of Claim 1 wherein $Z_1$, $Z_2$, and $Z_3$ are each H, comprising cultivating Trichoderma viride (ATCC 74084), or a mutant thereof, under conditions suitable for the formation of said compound and recovering the compound.